# EUROPEAN PATENT SPECIFICATION

(11) **EP 2 851 688 B1**
(45) Date of publication and mention of the grant of the patent: **05.06.2019**
(21) Application number: 13791453.7
(22) Date of filing: 01.04.2013
(51) Int. Cl.: G01N 33/68, G01N 33/574

(54) **Use of glycoprotein C4BPA as marker for detecting pancreatic cancer**
Verwendung der Glykoprotein C4BPA als Marker zum Nachweis von Bauchspeicheldrüsenkrebs
Utilisation de la glycoprotéine C4BPA comme marqueur de détection du cancer du pancréas

(30) Priority: 18.05.2012 JP 2012114048
(43) Date of publication of application: 25.03.2015
(73) Proprietor: Nitto Boseki Co., Ltd., Gonome Fukushima-shi Fukushima 960-8161 (JP)
(72) Inventor: NODA, Kenta, Koriyama-shi Fukushima 963-8061 (JP); MIURA, Toshihide, Koriyama-shi Fukushima 963-8061 (JP); NOMURA, Fumio, Chiba-shi Chiba 260-8670 (JP); SOGAWA, Kazuyuki, Chiba-shi Chiba 260-8670 (JP); SATO, Mamoru, Chiba-shi Chiba 260-8670 (JP); YOSHITOMI, Hideyuki, Chiba-shi Chiba 260-8670 (JP); MIYAZAKI, Masaru, Chiba-shi Chiba 260-8670 (JP)
(74) Representative: Hoffmann Eitle
(86) International application number: PCT/JP2013/059867
(87) International publication number: WO 2013/172105

(56) References cited:
- WO-A1-2012/100339
- MAKAWITA SHALINI ET AL: "Integrated proteomic profiling of cell line conditioned media and pancreatic juice for the identification of pancreatic cancer biomarkers.", MOLECULAR & CELLULAR PROTEOMICS : MCP OCT 2011, vol. 10, no. 10, October 2011 (2011-10), pages 1-21, XP002752273, ISSN: 1535-9484
- ODEGAARD O R ET AL: "Recurrent venous thrombosis during warfarin treatment related to acquired protein S deficiency.", THROMBOSIS RESEARCH 15 JUN 1992, vol. 66, no. 6, 15 June 1992 (1992-06-15), pages 729-734, XP002752274, ISSN: 0049-3848
- SHALINI MAKAWITA: 'Integrated Proteomic Profiling of Cell Line Conditioned Media and Pancreatic Juice for the Identification of Pancreatic Cancer Biomarkers' MOLECULAR & CELLULAR PROTEOMICS vol. 10, no. 10, 2011, XP055141194
- Sheng Pan ET AL: "Protein Alterations Associated with Pancreatic Cancer and Chronic Pancreatitis Found in Human Plasma using Global Quantitative Proteomics Profiling", JOURNAL OF PROTEOME RESEARCH., vol. 10, no. 5, 6 May 2011 (2011-05-06), pages 2359-2376, XP055393613, US ISSN: 1535-3893, DOI: 10.1021/pr101148r

## Description

### Technical Field

The present invention relates to the use of glycoprotein C4BPA as a marker for detecting pancreatic cancer "in vitro".

### Background Art

A malignant tumor occurring in the hepatobiliary pancreatic region, particularly pancreatic cancer, is known as a cancer with the poorest prognosis among digestive cancers, and this is mainly because such a cancer so easily infiltrates/metastasizes to surrounding organs that it has progressed to an advanced stage when diagnosed, and hence its resection rate is low, and more effective adjunctive therapy has not been established. Accordingly, in order to improve the efficiency of surgical resection, which is the only method expected to completely cure the cancer, it is urgently necessary to identify a specific diagnostic marker for pancreatic cancer, and it is significant to develop a molecular target drug taking the protein as a target, and to improve treatment outcome by performing an individualized treatment for attaining a higher therapeutic effect.

As a pancreatic cancer marker, CA19-9 is known (Non Patent Literature 1). Besides, it has been proposed to use a fucosylated oligosaccharide of human haptoglobin as a pancreatic cancer marker (Patent Literature 1). These markers are, however, not necessarily sufficient in their sensitivity and specificity, and it is desired to further develop a novel pancreatic cancer marker.

Makawita et al. (Molecular and Cellular Proteomics 2011, 10(10):1-21) describes AGR2, GLFM4, SYCN, COL6A1 and PIGR as pancreatic cancer biomarkers.

Odegaard et al. (Thrombosis Research 1992, 66(6):729-734) describes higher levels of mean total protein S and C4bPB concentrations in pancreatic carcinoma patients than in healthy controls.

Pan et al. (Journal of Proteome Research 2011, 10:2359-2376) describes protein alternations associated with pancreatic cancer and chronic pancreatitis in human plasma. C4BPA is identified as being differentially expressed in pancreatic cancer but not chronic pancreatitis samples.

### Citation List

### Patent Literature

Patent Literature 1: JP 2009-168470 A

### Non Patent Literature

Non Patent Literature 1: Br. J. Cancer, vol. 53, pp. 197-202

### Summary of Invention

### Technical Problem

Under these circumstances, an object of the present invention is to provide a method for detecting pancreatic cancer "in vitro" using glycoprotein C4BPA as marker, particularly, as a marker for detecting pancreatic cancer distinguishably from chronic pancreatitis or a healthy person.

### Solution to Problem

The present inventors have identified, for purpose of developing a novel pancreatic cancer marker excellent in terms of sensitivity and specificity, several cancer specific proteins in the hepatobiliary pancreatic region by using proteomics methods until now. With minimally invasive and highly versatile serums used as samples, pancreatic cancer specific glycoproteins in the serums were quantitatively identified by glycoprotein extraction and by using a TMT reagent (tandem mass tag reagent), resulting in finding a novel marker for detecting pancreatic cancer distinguishably from chronic pancreatitis or a healthy person, and thus, the present invention was established.

Accordingly, the present invention relates to:
[1] A method of detecting pancreatic cancer distinguishably from chronic pancreatitis, comprising measuring glycoprotein C4BPA contained in a sample and correlating an amount of the C4BPA with detection of pancreatic cancer, wherein the sample is serum, blood plasma, blood or urine.
[2] The method of detecting pancreatic cancer according to [1], comprising correlating an amount of CA19-9 in addition to the amount of the C4BPA, with the detection of pancreatic cancer.
[3] The method according to [1] or [2], wherein the glycoprotein C4BPA contained in a sample is measured by immunoassay, electrophoresis, mass spectrometry or liquid chromatography.
[4] The method of detecting pancreatic cancer according to any one of [1] to [3], which is a method for detecting pancreatic cancer distinguishably from chronic pancreatitis and a healthy person.
[5] Use of glycoprotein C4BPA as a marker for detecting pancreatic cancer distinguishably from chronic pancreatitis, wherein the use is not practiced on the human or animal body.
[6] The use according to [5], wherein the marker is used for detecting pancreatic cancer distinguishably from chronic pancreatitis and a healthy person.
[7] Use of a reagent kit comprising a reagent for measuring glycoprotein C4BPA for detecting pancreatic cancer distinguishably from chronic pancreatitis, wherein the use is not practiced on the human or animal body.
[8] The use according to [7], wherein said reagent is an antibody to the glycoprotein C4BPA.
[9] The use according to [7] or [8], for detecting pancreatic cancer distinguishably from chronic pancreatitis and a healthy person.

### Advantageous Effects of Invention

If glycoprotein C4BPA is used as a marker for detecting pancreatic cancer of the present invention, the possibility of pancreatic cancer can be specifically determined distinguishably from chronic pancreatitis patient and a healthy person. Besides, the prognostic state of a postoperative pancreatic cancer patient can be monitored. Furthermore, when it is used in combination with another pancreatic cancer marker, such as CA19-9, more specific determination can be made distinguishably from chronic pancreatitis patient and a healthy person.

### Brief Description of Drawings

Fig. 1 illustrates the results of a test by SDS-PAGE on proteins obtained from various serum samples.
Fig. 2 illustrates the results of measurement of C4BPA in various serum samples by an ELISA method.
Fig. 3 illustrates the results of measurement of PIGR in various serum samples by the ELISA method.
Fig. 4 illustrates ROC curves of C4BPA, PIGR and CA19-9 in diagnosis of pancreatic cancer.

### Description of Embodiments

The present invention will now be described in more details.

According to the present invention, as a result of quantitative identification of pancreatic cancer specific glycoproteins in serums by glycoprotein extraction and by using a TMT reagent performed on serum samples collected from healthy persons, chronic pancreatitis patients and pancreatic cancer patients (before and after operation), it was revealed that glycoprotein C4BPA is contained at a higher level in the serum samples obtained from the pancreatic cancer patients (before operation) as compared with that in the serum samples obtained from the healthy persons, the chronic pancreatitis patients and the pancreatic cancer patients (after operation), and accordingly, it was found that the C4BPA can be a marker for pancreatic cancer.

The C4BPA, namely, C4b-binding protein alpha chain, is a glycoprotein having UniProt accession number P04003 (http://www.uniprot.org/uniprot/P04003). The C4BPA controls the classical pathway of complement activation. The C4BPA is a cofactor of C3bINA and hydrolyses a complement fragment C4b. Besides, it has a function to accelerate decomposition of a C4bC2a complex and a complement fragment C2a (Barbosa et al., Infection and Immunity, Mr. 2009, pp. 1137-1143; and Dahlback et al., Proc. Natl. Acad. Sci. USA Vol. 80, pp. 3461-3465, June 1983).

On the other hand, the PIGR, namely, a polymeric immunoglobulin receptor, is a glycoprotein having UniProt accession number P01833 (http://www.uniprot.org/uniprot/P01833). The PIGR binds to IgA and IgM polymers on the outside surface of the bases of epithelial cells. A complex resulting from the binding is transported to the outside of the cells through a transmembrane region during the cleavage process (Deitcher et al., The Journal of Cell Biology, Volume 102, March 1986, 911-919; and Asano et al., Journal of Oral Science, Vol. 53, No. 2, 147-156, 2011).

In particular, using C4BPA as marker, pancreatic cancer can be detected distinguishably from chronic pancreatitis or a healthy person.

In a measurement method of the present invention, the C4BPA contained in a sample is measured for detecting pancreatic cancer on the basis of the C4BPA.

In the present invention, the C4BPA contained in a sample is measured, and the amount of the C4BPA is correlated with detection of pancreatic cancer, and thus, pancreatic cancer can be detected. In actually measuring the C4BPA contained in a sample, the C4BPA as a glycoprotein may be measured, or an amino acid sequence portion of the C4BPA obtained by, for example, removing an oligosaccharide from the glycoprotein through sample processing or the like may be measured.

In the present invention, for example, the level of the C4BPA contained in a sample collected from a patient suspected of having pancreatic cancer or a postoperative pancreatic cancer patient is measured, so as to determine possibility of developing pancreatic cancer or a prognostic state. In this case, if the level of the C4BPA is higher than that of a healthy person, it can be determined that there is a high possibility that pancreatic cancer is detected, and alternatively, if the level of the C4BPA is lowered in a sample obtained from a postoperative pancreatic cancer patient, it can be determined that the prognosis of the pancreatic cancer is good.

In the present invention, pancreatic cancer can be more specifically detected distinguishably from chronic pancreatitis patient or a healthy person by using another pancreatic marker, such as CA19-9, in combination.

Examples of the sample usable in the present invention include serum, blood plasma, blood and urine collected from a patient suspected of having pancreatic cancer or a postoperative pancreatic cancer patient.

In order to measure the C4BPA, any of currently known methods can be employed. Examples of the methods include immunoassay, electrophoresis, mass spectrometry and liquid chromatography.

Examples of the immunoassay include an immunoturbidimetric assay and an enzyme-linked immunoassay. The immunoassay can be performed by using, as an antibody, a polyclonal antibody or a monoclonal antibody corresponding to an anti-C4BPA antibody for performing measurement by using a conventionally known protein as an antigen. As the anti-C4BPA antibody, a commercially available antibody can be used, or such an antibody can be produced by a known method. Such an antibody may specifically recognize the structure of the amino acid sequence of the C4BPA or the PIGR, or may specifically recognize the whole structure thereof including the sugar chain.

The immunoturbidimetric assay is not especially limited as long as it is performed by causing an antigen-antibody reaction between the C4BPA contained in a sample and the anti-C4BPA antibody, and measuring the concentration of the C4BPA on the basis of the degree of turbidity resulting from the reaction. Examples of such a method include a TIA method, latex immunoturbidimetry and a nephelometry method. In the TIA method, the degree of turbidity is measured based on an absorbance at, for example, 340 nm to 800 nm in the immunoturbidimetric assay. In the latex immunoturbidimetry, the measurement is performed by using, as an antibody, the anti-C4BPA antibody bound to a latex particle in the immunoturbidimetric assay. In the nephelometry method, the degree of turbidity is measured using scattering light obtained by collecting light scattered at an angle not less than a prescribed angle in the immunoturbidimetric assay.

An example of the enzyme-linked immunoassay includes an EIA method using a plate as a support. This method will now be specifically described. First, to a solid known as itself of polystyrene, polypropylene, polycarbonate, polyethylene, nylon, polymethacrylate or the like, an anti-C4BPA antibody is directly or indirectly bound as a primary antibody by using a physical bond, a chemical bond or an affinity. The amount of sensitizing antibody is generally, for example, in a range of 1 ng to 100 mg/ml.

In the case where the C4BPA is to be measured by the enzyme-linked immunoassay, to the primary antibody bound to the solid by using, for example, a physical bond, a chemical bond or an affinity, a sample for measuring the C4BPA is added to cause a reaction. After performing the reaction for a prescribed time period, the solid is washed, and a secondary labeled antibody is added thereto to cause a secondary reaction. The solid is washed again, and a labeled portion bound to the solid is measured.

In the immunoassay described above, a protein that specifically recognizes and binds/crosslinks with the oligosaccharide of the C4BPA, namely, a lectin corresponding to a specific binding partner, can be used instead of the anti-C4BPA antibody. As the lectin, a lectin specifically recognizing fucose is preferably used, and an example includes an aleuria aurantia lectin (AAL) that binds to an oligosaccharide having L-fucose. In this case, for example, the lectin and the anti-C4BPA antibody may be used and they are combined with either a specific binding partner bound to a solid or a labeled specific binding partner, respectively, so that the measurement can be performed by a sandwich method.

In the immunoassay using an antibody or a specific partner such as a lectin, an enzyme such as a horseradish peroxidase (HRP) alkaline phosphatase can be used as a labeling substance. For example, if an HRP labeled antibody is used, known DAB, TMB, OPD or the like can be used as a substrate. Besides, the labeling substance is not limited to an enzyme such as HRP but can be any substances capable of labeling, including a labeling metal such as gold colloid or europium, chemical or biological various fluorescent substances such as FITC, rhodamine, Texas Red, Alexa and GFP, and radioactive substances such as ³²P and ⁵¹Cr. Besides, if a labeling substance is used in the present invention, an avidin-biotin system or a streptavidin-biotin system can be used.

As the electrophoresis, an SDS-PAGE method can be generally employed. In the SDS-PAGE method, a protein is electrophoresed in a gel of polyacrylamide, and since the mobility in a prescribed time period and the number of amino acids constituting the protein, namely, the molecular weight thereof, are in proportion to each other, the protein is thus classified. In a similar method, cellulose acetate may be used as a support. For staining a protein, Coomassie brilliant blue, Ponceau S stain, amido black stain or direct enzyme activity is utilized. For example, a prescribed amount of a sample and a general sample buffer are mixed in a prescribed ratio of 1:1, and the resultant is heated. In general, the resultant mixture is treated in boiling water for about 5 minutes. Thereafter, the sample is applied to a gel. In polyacrylamide electrophoresis, a good result can be obtained when the protein is run at a low current. In general, a current of about 5 to 100 mA is used. After the electrophoresis, the resultant is stained with the above-described reagent, and treated with a bleaching liquid containing a mixture of acetic acid, methanol and water, and thus, a band of the C4BPA can be confirmed. The level of the C4BPA can be measured depending on the intensity of the band.

Besides, detection by a western blotting method is also effective. Specifically, a gel resulting from the electrophoresis is transferred to a nitrocellulose membrane, a PVDF membrane or the like, then reacted with a primary antibody of an anti-C4BPA antibody or an anti-PIGR antibody and further with a secondary labeled antibody of an HPR labeled anti-IgG, and then colored with an HRP coloring reagent (Wako), and on the basis of the degree of coloring of a band corresponding to the C4BPA, the C4BPA can be measured.

As the mass spectrometry, an analysis method using a mass spectrograph, which has been recently rapidly developed, can be employed. Examples of such a method include a method using a surface enhanced laser desorption/ionization time-of-flight mass spectrometer (a SELDI-TOF MS method), a method using a matrix-assisted laser desorption/ionization time-of-flight mass spectrometer (a MALDI-TOF MS method), and a method using an ESI (electrospray ionization) method. In the SELDI-TOF MS method, with a target substance uniformly captured by functional groups on the surface of a chip, impurities are removed and ionization is performed by a laser beam, so that a reproducible ion spectrum with a high S/N ratio can be obtained, and hence this method is preferable.

In employing the SELDI-TOF MS method, in general, after a pretreatment of a sample, the resulting sample is adsorbed onto a chip to be loaded in the SELDI-TOF MS mass spectrometer. If the sample is serum, it is preferred to use an albumin absorbent or to remove albumin from the system through washing with a buffer by using an ion exchange chip until albumin has no charge. As a chip used in the SELDI-TOF MS method for binding a protein, the type of chip is not especially limited as long as it can adsorb a protein such as the C4BPA or the PIGR. Examples include a chip modified with a functional group having an affinity with a hydrophobic or ion exchange protein, etc. (that is also designated as a chemical chip) and a chip on which an antibody to the C4BPA is immobilized (biochemical chip).

In the MALDI-TOF MS method, a sample is mixed with a compound absorbing a laser beam, and the resultant mixture is irradiated with a laser beam of a short period of time of several nanoseconds to ionize a protein contained in the sample for measuring the protein. As a chip for binding the protein, chips similar to those used in the SELDI-TOF MS method can be used. In the ESI method or the like, a sample having been subjected to a pretreatment such as a protease treatment is preferably loaded in a mass spectrometer directly connected to separation means such as high performance liquid chromatography.

In preferable embodiments, separately detectable isotope-labeled C4BPA is added to a sample as an internal standard substance. In these embodiments, the whole or a part of both intrinsic C4BPA present in the sample and the internal standard substance is ionized to generate a plurality of ions detectable by a mass spectrometer, and one or more ions generated from each of them are detected by the mass spectrometry. In a related embodiment, the isotope-labeled C4BPA may contain ¹³C and ¹⁵N isotope-labeled valine, arginine, isoleucine, leucine, lysine, phenylalanine, proline subunits or combinations of these. In further preferable embodiments, in the isotope-labeled C4BPA, a carbon atom is substituted with a ¹³C isotope or a nitrogen atom is substituted with a ¹⁵N isotope, and the intrinsic C4BPA increases the mass as compared with natural C4BPA. In preferable related embodiments, the isotope labeling has an amino acid subunit in which some of or all the carbon atoms are substituted with the ¹³C isotope and some of or all the nitrogen atoms are substituted with the ¹⁵N isotope. The mass of the isotope-labeled C4BPA is generally larger than that of natural one.

In preferable embodiments, the presence or absence and/or the amount of C4BPA ions is correlated with their amount in a sample through comparison with the internal standard substance.

The liquid chromatography is a method known to those skilled in the art, and such a known method can be employed in the present invention. An example of the liquid chromatography includes one employing an affinity column using, as a support, lectin that is a protein specifically recognizing and binding/crosslinking with the oligosaccharide of the C4BPA, such as the aleuria aurantia lectin (AAL) binding to an oligosaccharide having L-fucose.

use of a The present invention also provides a use of a reagent kit for detecting pancreatic cancer "in vitro" wherein the kit contains a reagent for measuring the glycoprotein C4BPA for performing the aforementioned various assays. Such a kit can contain, as a composing element, any of various reagents in accordance with the employed assay. For performing the immunoassay, for example, an antibody to the glycoprotein C4BPA can be contained. As such an antibody, a commercially available antibody can be used, or it can be produced by a known method. Such an antibody may specifically recognize the structure of the amino acid sequence of the C4BPA, or may specifically recognize the whole structure thereof including the sugar chain. The kit may further contain any of known reagents such as a coloring reagent for a labeling substance in accordance with an assay to be performed. Examples

The present invention will now be specifically described with reference to preferable examples, and it is noted that the present invention is not limited to these examples. Examples showing measurements of PIGR as a marker for detecting pancreatic cancer are also shown as comparative examples.

### Example 1

### Identification of pancreatic cancer marker

Serum samples collected from 45 healthy persons, 25 chronic pancreatitis patients and 57 pancreatic cancer patients were used for searching and identifying a pancreatic cancer marker.

### A. Method

### 1. Serums to be examined

Serum samples collected from 45 healthy persons, 25 chronic pancreatitis patients and 57 pancreatic cancer patients were used as serum for searching and identifying a pancreatic cancer marker.

### 2. Extraction of glycoproteins from each pool serum

Three kinds of pool serums were obtained by extracting and pooling the serums of randomly selected five each of healthy persons, chronic pancreatitis patients and pancreatic cancer patients (before and after operation).

Glycoproteins were extracted from each pool serum by using VEC TREX AAL Binding and Elution Kit (Vector Laboratories), that is, a glycoprotein search kit using a fucose-specific lectin, the aleuria aurantia lectin (AAL) binding to an oligosaccharide having L-fucose.

20 µL of the pool serum was put in 20 µL of AAL beads, and the resultant was incubated for 1 hour and then centrifuged at 12000 xg for 1 minute, and a supernatant was discarded. To the remaining, 80 µL of TENT buffer (100 mM Tris, pH 8.0; 10 mM EDTA; 1.5M NaCl; 1.0% (v/v) Tween 20) was added and mixed, the resultant mixture was then centrifuged at 12000 xg for 1 minute, and a supernatant was discarded. To the remaining, 20 µL of Elution buffer (1.25 M L-fucose; TENT buffer) was added and mixed, the resultant mixture was then incubated at room temperature for 1 hour, and centrifuged at 12000 xg for 1 minute, and then, a supernatant was collected, and thus glycoproteins were extracted from each pool serum to obtain three kinds of samples to be labeled.

### 3. Labeling of glycoproteins

The samples to be labeled were labeled by using TMT Mass Tagging Kits and Reagents (Thermo Scientific Inc.).

In this method using a TMT (tandem mass tag) reagent, specific glycoproteins contained in each pool serum can be tagged to have same physical properties but different molecular weights. As a result, when this method is employed, the abundance ratios of the specific glycoproteins in each sample can be quantified by mixing a plurality of labeled samples. Specific labeling of the glycoproteins was performed as follows:
20 µL of each sample to be labeled obtained as described in section 2 above (corresponding to 20 µL of serum) was treated with AAL (aleuria aurantia lectin) beads, subsequently, 60 µL of 50 mM TEAB (triethyl ammonium bicarbonate), 5 µL of 2% SDS and 5 µL of 200 mM TCEP (tris(2-carboxyethyl)phosphine) were added to the resultant, followed by incubation at 55°C for 1 hour, and 5 µL of 375 mM iodoacetamide was further added thereto, and the resultant was allowed to stand still at room temperature for 30 minutes. Thereafter, 40 µL of the TMT reagent in each mass having been solubilized with 42 µL of 100% acetonitrile was added to each of the resultant samples, followed by incubation at room temperature for 1 hour, and 8 µL of 5% hydroxylamine was further added thereto, and the resultant was allowed to stand still at room temperature for 15 minutes to terminate the reaction. Thereafter, each sample was washed with cooled acetone and freeze-dried again, and thus, labeled samples of the glycoproteins were obtained.

### 4. Identification of proteins by SDS-PAGE

In the SDS-PAGE, 10-20% gradient gel (DRC) was used. On lane 1, a sample of a mixture of labeled glycoproteins obtained by pretreating the healthy person pool serum, the chronic pancreatitis patient pool serum and the pancreatic cancer patient pool serum was run, and on lane 2, a sample of a mixture of labeled glycoproteins obtained by pretreating the healthy person pool serum, the pancreatic cancer patient (before operation), and the pancreatic cancer patient (after operation) pool serum was run. Incidentally, in lane 1, TMT-126, TMT-127 and TMT-128 tags were respectively used as mass reporters for the samples derived from the healthy persons, the chronic pancreatitis patients and the pancreatic cancer patients (before operation). Besides, in lane 2, TMT-126, TMT-127 and TMT-128 tags were respectively used for the samples derived from the healthy person pool serum, the pancreatic cancer patient (before operation), and the pancreatic cancer patient (after operation).

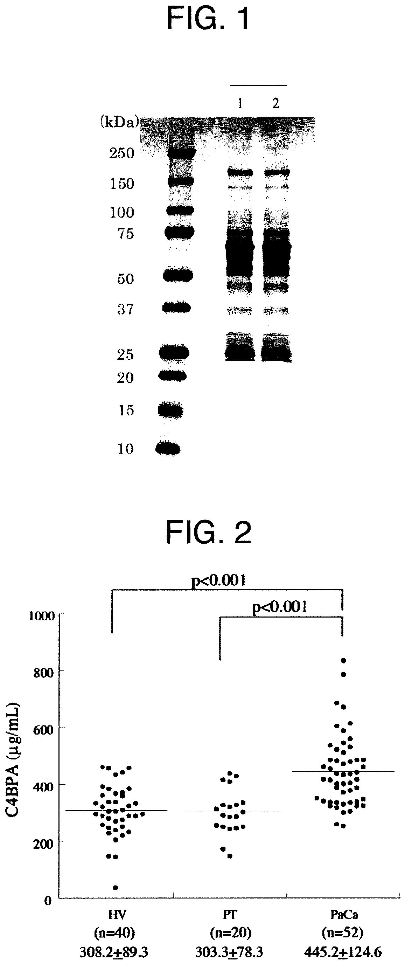

A gel resulting from the electrophoresis was Coomassie stained, and protein bands were detected (Fig. 1). Each lane was divided into eighteen sections and cut out of the gel for performing in-gel digestion with trypsin. The resulting digested fragments were separated by using HPLC, followed by MS/MS measurement by LTQ Orbitrap XL (Thermo Scientific Inc.). The obtained measurement data was subjected to database search by Mascot search engine (Matrix Science) for identifying proteins. Quantitative comparison between the proteins of the respective pool serums was performed by using Proteome Discoverer (Thermo Scientific Inc.).

### B. Results

The results of the quantitative comparison between the abundances of proteins in the samples obtained by using a mass spectrometer are shown in Table 1.

### [Table 1]

**Table 1**

| UniProt Accession No. | Marker | Mass | Ratio between PaCa and HV | Ratio between PT and HV | Ratio between pre OPE and HV | Ratio between post OPE and HV |
|---|---|---|---|---|---|---|
| P04003 | C4BPA | 67,033 | 2.47 | 1.11 | 3.46 | 1.14 |
| P01833 | PIGR | 83,284 | 2.18 | 0.83 | 3.68 | 1.45 |
| P14780 | Matrix metalloproteinase-9 | 78,458 | 1.64 | 0.83 | not detected | not detected |
| Q06033 | Inter-alpha-trypsin inhibitor heavy chain H3 | 99,849 | 2.67 | 1.44 | not detected | not detected |

| | | | | | | |
|---|---|---|---|---|---|---|
| Pancreatic cancer pool serum: PaCa, Chronic Pancreatitis patient pool serum: PT Healthy person pool serum: HV Pancreatic cancer patient pool serum (before operation): pre OPE, Pancreatic cancer patient pool serum (after operation): post OPE | | | | | | |

On the basis of the results shown in Table 1, proteins having a ratio between the pancreatic cancer patient pool serum (before operation) and the healthy person pool serum of 1.5 or more, having a ratio between the pancreatic cancer patient pool serum (after operation) and the healthy person pool serum of 1.5 or less, having a ratio between the chronic pancreatitis patient pool serum and the healthy person pool serum of 1.5 or less and a ratio between the pancreatic cancer patient pool serum and the healthy person pool serum of 1.5 or more were screened to identify the C4BPA and the PIGR.

Particularly good results were obtained in that the C4BPA and the PIGR distinguished, as a pancreatic cancer marker, a pancreatic cancer (preoperative) patient from a healthy person, a chronic pancreatitis patient and a pancreatic cancer (postoperative) patient, that a difference in data between the pancreatic cancer patients after operation and the heathy persons was small, and that a difference in data between the pancreatic cancer patients before operation and the healthy persons was large, namely, that there was a large difference in data of the pancreatic cancer patients between before and after operation, and these results reveal that the C4BPA and the PIGR are suitably used as pancreatic cancer markers.

On the other hand, with respect to Matrix metalloproteinase-9 and Inter-alpha-trypsin inhibitor heavy chain H3, although there was a difference in data between the healthy persons and the pancreatic cancer patients, there was no difference in data between before and after operation in the screening performed on the aforementioned basis, and they were excluded from marker candidates.

### Example 3

### Usefulness of C4BPA and PIGR as pancreatic cancer marker

The usefulness of the C4BPA and the PIGR as a pancreatic cancer marker was verified through measurement of the C4BPA or the PIGR in a sample by an ELISA method using an anti-C4BPA antibody or anti-PIGR antibody. Besides, the usefulness of the C4BPA and the PIGR as the pancreatic cancer marker was verified also through evaluation based on an ROC curve generally frequently performed for accuracy evaluation of a diagnostic test or a screening test and for comparison with a conventional method.

### 1. Measurement of C4BPA or PIGR by ELISA

### (1) Method

With respect to the C4BPA and the PIGR having showed particularly good data as a pancreatic cancer marker, ELISA measurement was performed using various samples. The C4BPA and the PIGR were measured using C4 Binding Protein α ELISA Kit (Uscn Life Sciences & Technology Inc.) and Polymeric Immunoglobulin Receptor ELISA Kit (Uscn Life Sciences & Technology Inc.), respectively.

The measurement using the ELISA kit was performed according to the manufacturer's protocol. Specifically, an anti-C4BPA antibody or an anti-PIGR antibody was immobilized as a primary antibody on a microtiter plate, and a diluted sample was added to the antibody, followed by incubation. Subsequently, after washing the plate, an HRP labeled antiC4BPA antibody or anti-PIGR antibody was added thereto for causing a reaction, followed by incubation. After washing the plate again, TMB was added thereto to cause a coloring reaction, and after adding a reaction terminator, the measurement was performed at 450 nm by using iMark TM Microplat Reader (Bio-Rad Laboratories, Inc.). The thus obtained data was compared with a precedently prepared calibration curve, so as to measure the C4BPA or the PIGR contained in the sample. For statistical processing, IBM SPSS Statistics 18 software (SPSS Inc.) was used. A two-sample test was evaluated by using the Mann-Whitney U-test, and it was determined that there was a significant difference if p < 0.05.

### (2) Results

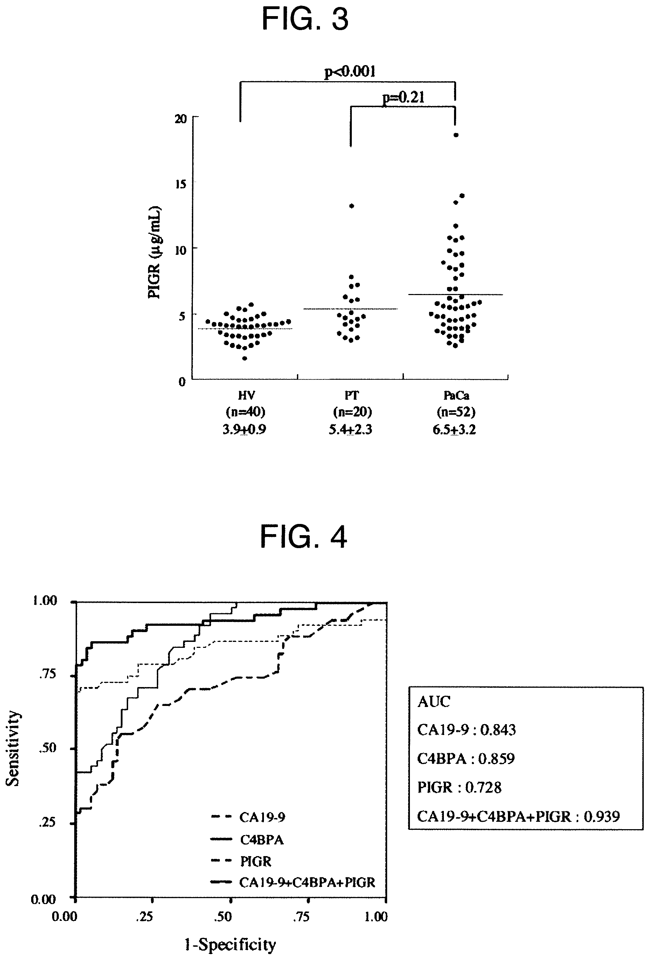

The results for the C4BPA and the PIGR are shown in Figs. 2 and 3, respectively.

It is understood from the results shown in Fig. 2 that the C4BPA was present in the samples from the healthy persons (HV), the chronic pancreatitis patients (PT) and the pancreatic cancer patients (before operation) (PaCa) respectively in 308.2 ± 89.3 µg/mL, 303.3 ± 78.3 µg/mL, and 445.2 ± 124.6 µg/mL, and p < 0.001, and hence there was a significant difference between the samples from the pancreatic cancer patients and the samples from the healthy persons and the chronic pancreatitis patients. Accordingly, it was revealed that the C4BPA is useful as pancreatic cancer marker for distinguishing pancreatic cancer (preoperative) patient from a healthy person or chronic pancreatitis patient.

It is understood from the results shown in Fig. 3 that the PIGR was present in the samples from the healthy persons (HV), the chronic pancreatitis patients (PT) and the pancreatic cancer patients (PaCa) respectively in 3.9 ± 0.9 µg/mL, 5.4 ± 2.3 µg/mL, and 6.5 ± 3.2 µg/mL, and p < 0.001, and hence there was a significant difference between the samples from the pancreatic cancer patients from the samples from the healthy persons. Accordingly, it was revealed that the PIGR is useful as pancreatic cancer marker for distinguishing pancreatic cancer (preoperative) patient from a healthy person.

### 2. Evaluation of C4BPA and PIGR as pancreatic cancer marker based on ROC curve

The ROC curves of the C4BPA and the PIGR in diagnosis of pancreatic cancer are shown in Fig. 4 together with the ROC curve of CA19-9 conventionally used for the diagnosis of pancreatic cancer. The ROC curve was obtained by using statistical analysis software SPSS ver. 18 with a cutoff value calculated based on the 2SD of the concentration in a healthy person of each marker. AUROC (area under the receiver operating characteristics curves) obtained from the ROC curves were 0.843 for the CA19-9, 0.859 for the C4BPA and 0.728 for the PIGR. Besides, when the CA19-9, the C4BPA and the PIGR were combined, AUROC was 0.939. Furthermore, when the CA19-9 and the C4BPA were combined, AUROC was 0.929 (not shown). Accordingly, it was revealed that the measurement results of the C4BPA and the PIGR are useful for the diagnosis of pancreatic cancer, and it was found that it is further useful for the diagnosis of pancreatic cancer to make determination by combining the measurement result of the CA19-9 with the measurement result of the C4BPA or the PIGR.

### Industrial Applicability

As described in detail so far, if the glycoprotein C4BPA or PIGR is used as a marker for detecting pancreatic cancer, the possibility of pancreatic cancer can be specifically determined distinguishably from chronic pancreatitis patient and a healthy person. Besides, the prognostic state of a postoperative pancreatic cancer patient can be monitored. Furthermore, if combined with another pancreatic cancer marker, such as CA19-9, more specific determination can be made distinguishably from chronic pancreatitis patient and a healthy person.

## Claims

1. A method of detecting pancreatic cancer distinguishably from chronic pancreatitis, comprising measuring glycoprotein C4BPA contained in a sample and correlating an amount of the C4BPA with detection of pancreatic cancer, wherein the sample is serum, blood plasma, blood or urine.

2. The method of detecting pancreatic cancer according to claim 1, comprising correlating an amount of CA19-9 in addition to the amount of the C4BPA, with the detection of pancreatic cancer.

3. The method according to claim 1 or 2, wherein the glycoprotein C4BPA contained in a sample is measured by immunoassay, electrophoresis, mass spectrometry or liquid chromatography.

4. The method of detecting pancreatic cancer according to any one of claims 1 to 3, which is a method for detecting pancreatic cancer distinguishably from chronic pancreatitis and a healthy person.

5. Use of glycoprotein C4BPA as a marker for detecting pancreatic cancer distinguishably from chronic pancreatitis, wherein the use is not practiced on the human or animal body.

6. The use according to claim 5, wherein the marker is used for detecting pancreatic cancer distinguishably from chronic pancreatitis and a healthy person.

7. Use of a reagent kit comprising a reagent for measuring glycoprotein C4BPA for detecting pancreatic cancer distinguishably from chronic pancreatitis, wherein the use is not practiced on the human or animal body.

8. The use according to claim 7, wherein said reagent is an antibody to the glycoprotein C4BPA.

9. The use according to claim 7 or 8, for detecting pancreatic cancer distinguishably from chronic pancreatitis and a healthy person.

## Patentansprüche

1. Verfahren zum von chronischer Pankreatitis unterscheidbaren Detektieren von Pankreaskrebs, umfassend das Messen von Glycoprotein C4BPA, enthalten in einer Probe, und Korrelieren einer Menge des C4BPA mit der Detektion von Pankreaskrebs, wobei die Probe Serum, Blutplasma, Blut oder Urin ist.

2. Verfahren zum Detektieren von Pankreaskrebs gemäß Anspruch 1, umfassend das Korrelieren einer Menge von CA19-9 zusätzlich zur Menge des C4BPA mit der Detektion von Pankreaskrebs.

3. Verfahren gemäß Anspruch 1 oder 2, wobei das in einer Probe enthaltene Glycoprotein C4BPA durch Immunassay, Elektrophorese, Massenspektrometrie oder Flüssigchromatographie gemessen wird.

4. Verfahren zum Detektieren von Pankreaskrebs gemäß irgendeinem der Ansprüche 1 bis 3, welches ein Verfahren zum von chronischer Pankreatitis und einer gesunden Person unterscheidbaren Detektieren von Pankreaskrebs ist.

5. Verwendung des Glycoproteins C4BPA als ein Marker zum von chronischer Pankreatitis unterscheidbaren Detektieren von Pankreaskrebs, wobei die Verwendung nicht am menschlichen oder tierischen Körper ausgeführt wird.

6. Verwendung gemäß Anspruch 5, wobei der Marker zum von chronischer Pankreatitis und einer gesunden Person unterscheidbaren Detektieren von Pankreaskrebs verwendet wird.

7. Verwendung eines Reagenskits, umfassend ein Reagens zum Messen des Glycoproteins C4BPA zum von chronischer Pankreatitis unterscheidbaren Detektieren von Pankreaskrebs, wobei die Verwendung nicht am menschlichen oder tierischen Körper ausgeführt wird.

8. Verwendung gemäß Anspruch 7, wobei das Reagens ein Antikörper gegen das Glycoprotein C4BPA ist.

9. Verwendung gemäß Anspruch 7 oder 8 zum von chronischer Pankreatitis und einer gesunden Person unterscheidbaren Detektieren von Pankreaskrebs.

## Revendications

1. Procédé de détection du cancer du pancréas de manière différenciable d'une pancréatite chronique, comprenant
la mesure de la glycoprotéine C4BPA contenue dans un échantillon et la corrélation d'une quantité de la C4BPA avec la détection du cancer du pancréas, où l'échantillon est du sérum, du plasma sanguin, du sang ou de l'urine.

2. Procédé de détection du cancer du pancréas selon la revendication 1, comprenant la corrélation d'une quantité de CA19-9 en plus de la quantité de la C4BPA, avec la détection du cancer du pancréas.

3. Procédé selon la revendication 1 ou 2, dans lequel la glycoprotéine C4BPA contenue dans un échantillon est mesurée par un dosage immunologique, une électrophorèse, une spectrométrie de masse ou une chromatographie en phase liquide.

4. Procédé de détection du cancer du pancréas selon l'une quelconque des revendications 1 à 3, qui est un procédé de détection du cancer du pancréas de manière différenciable d'une pancréatite chronique et d'un individu en bonne santé.

5. Utilisation de la glycoprotéine C4BPA en tant que marqueur pour la détection du cancer du pancréas de manière différenciable d'une pancréatite chronique, dans laquelle l'utilisation n'est pas pratiquée sur le corps humain ou animal.

6. Utilisation selon la revendication 5, dans laquelle le marqueur est utilisé pour détecter le cancer du pancréas de manière différenciable d'une pancréatite chronique et d'un individu en bonne santé.

7. Utilisation d'un kit de réactif comprenant un réactif pour mesurer la glycoprotéine C4BPA afin de détecter le cancer du pancréas de manière différenciable d'une pancréatite chronique, dans laquelle l'utilisation n'est pas pratiquée sur le corps humain ou animal.

8. Utilisation selon la revendication 7, dans laquelle ledit réactif est un anticorps dirigé contre la glycoprotéine C4BPA.

9. Utilisation selon la revendication 7 ou 8, pour la détection du cancer du pancréas de manière différenciable d'une pancréatite chronique et d'un individu en bonne santé.
